(19) Europäisches Patentamt / European Patent Office / Office européen des brevets

(11) **EP 4 726 052 A1**

(12) **EUROPEAN PATENT APPLICATION**

(43) Date of publication:
**15.04.2026  Bulletin 2026/16**

(21) Application number: 25199419.0

(22) Date of filing: **01.09.2025**

(51) International Patent Classification (IPC):
*C12P 21/02* (2006.01)   *C12N 9/08* (2006.01)
*C12M 1/00* (2006.01)   *C12M 1/06* (2006.01)
*C12M 1/26* (2006.01)   *C12M 1/34* (2006.01)
*C12Q 3/00* (2006.01)

(52) Cooperative Patent Classification (CPC):
**C12P 21/02; C12N 9/0089; C12Y 115/01001;**
**C12M 21/14**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **11.10.2024  CN 202411415571**

(71) Applicant: **Liaoning Future Biotech Co., Ltd.
Benxi Liaoning 117004 (CN)**

(72) Inventors:
• ZHAO, Xiaoli
  BENXI 117004 (CN)
• WANG, Jianying
  BENXI 117004 (CN)
• MA, Jiapeng
  BENXI 117004 (CN)
• WEI, Lifu
  BENXI 117004 (CN)

(74) Representative: **Von Rohr Patentanwälte
Partnerschaft mbB
Rüttenscheider Straße 62
45130 Essen (DE)**

(54) **PREPARATION METHOD FOR CORN SUPEROXIDE DISMUTASE**

(57)    The present invention discloses a preparation method for corn superoxide dismutase, relating to the technical field of organic matter separation and extraction, including: S1. corn pretreatment; S2. enzymatic hydrolysis: S21. transferring a corn slurry into a rotary screen, and immersing a lower half of a drum into an enzymatic hydrolysis mixed liquid; starting the rotary screen, so as to enable a corn material to tumble inside the drum while remaining immersed in the enzymatic hydrolysis mixed liquid; S22. extracting the enzymatic hydrolysis mixed liquid at fixed intervals to undergo centrifugal separation, so as to remove starch granules and other impurities; S23. pouring a centrifuged clear liquid back into the rotary screen, and performing enzymatic hydrolysis cyclically; S24. uniformly stirring the corn slurry having undergone the enzymatic hydrolysis and filtering, collecting a filtrate; and S3. extraction and post-treatment, thus solving the problem of viscosity caused by the high starch content when using corn as the raw material, significantly improving efficiency of enzymatic hydrolysis, effectively protecting activity of SOD, and rendering stable and reliable product quality; at the same time, the technical solution facilitates large-scale production and reduces production costs.

Rotary screen

Enzymatic hydrolysis mixed liquid

Liquid surface

Material

FIG. 1

EP 4 726 052 A1

# Description

## TECHNICAL FIELD

The present invention relates to the technical field of organic matter separation and extraction, and particularly to a preparation method for corn (*Zea mays L.*) superoxide dismutase.

## BACKGROUND ART

Superoxide dismutase (SOD) is an antioxidant metalloenzyme existing in organisms, which can catalyze dismutation of superoxide anion radicals to generate oxygen and hydrogen peroxide, plays a vital role in the balance of oxidation and anti-oxidation of organisms, and is inextricably linked to pathogenesis and progression of many diseases.

SOD is extracted from sources such as microorganisms such as yeast, bacteria, fungi, and bioengineered bacteria and roots and stems, leaves, seeds or other fruits of plants. Currently, extracting SOD from plants is a common method, mostly from garlic, corn, tomato, mulberry leaf, seabuckthorn fruit, cactus and other plants.

For example, a Chinese patent with filing No. CN202011634220.1 discloses an industrial production method for extracting superoxide dismutase from Shenzhou grass. This invention provides an industrial production method for extracting superoxide dismutase from Shenzhou grass, pertaining to the technical field of organic matter separation and extraction. The method includes: S1. uniformly mixing a Shenzhou grass raw material with a phosphate buffer solution, and then adding an macerating enzyme and a surfactant to perform an enzymatic hydrolysis treatment; S2. after the enzymatic hydrolysis, performing ultrasonic extraction and solid-liquid separation to obtain a liquid; and S3. adding any one or more of an activator, a protective agent and a stabilizer into the liquid, filtering and collecting Shenzhou grass superoxide dismutase. This invention aims to be suitable for industrial production, and by optimizing various process steps for extracting superoxide dismutase from Shenzhou grass, an industrial production method for extracting superoxide dismutase from Shenzhou grass is finally obtained, with a good practical application value.

Compared with Shenzhou grass, a corn raw material is more accessible. There are currently many methods for preparing superoxide dismutase complex using corn. However, there exists a problem of using a chemical disinfectant such as bleach powder and sodium hypochlorite in a preparation process. As the chemical disinfectants are prone to leave residues, and cause environmental pollution during production process, quality of the prepared superoxide dismutase complex is also adversely affected. At the same time, in the process of drying a product, the existing methods use freeze drying or spraying, the former has a relatively high cost, and the latter has a relatively large loss of enzyme activity, both are not suitable for large-scale production. The existing methods also have technical problems of low germination rate, low enzyme yield, small production scale, etc. Germination of corn within a chamber of a germination apparatus suffers from a problem of small ventilation volume and a confined growing environment, a bacterial biofilm tends to be formed on corn surfaces, causing putrefaction phenomena, reducing a germination rate, and degrading quality of prepared superoxide dismutase, moreover, it is prone to residue and difficult to clean, thus increasing workers' labor intensity.

In addition, high starch content in corn results in a viscous liquid during enzymatic hydrolysis, which hinders contact of enzyme with cell walls, reduces efficiency of enzymatic hydrolysis, and affects activity and extraction amount of SOD.

## SUMMARY

Embodiments of the present invention provide a preparation method for a corn superoxide dismutase, which solves the problem of viscosity caused by high starch content during SOD production with corn as a raw material, significantly improves efficiency of enzymatic hydrolysis, effectively protects activity of SOD, and renders stable and reliable product quality. Moreover, the technical solution facilitates large-scale production and reduces production costs.

Embodiments of the present invention provide a preparation method for a corn superoxide dismutase, specifically including following steps:

S1. pre-treatment: pulverizing sprouted corn to 80-120 mesh, into the pulverized sprouted corn, adding a pH=7.8, 0.05 mol/L phosphate-buffered saline at a ratio of 1:2-4, and mixing uniformly and grinding to obtain a corn slurry, followed by ultrasonic cell disruption;

S2. enzymatic hydrolysis:

S21. transferring the corn slurry having undergone ultrasonic disruption into a rotary screen, and immersing a lower half of a drum into an enzymatic hydrolysis mixed liquid; starting the rotary screen, so as to enable a corn material to tumble inside the drum while remaining immersed in the enzymatic hydrolysis mixed liquid;

S22. extracting the enzymatic hydrolysis mixed liquid every 10-15 minutes to undergo centrifugal separation, so as to remove suspended starch granules and other impurities;

S23. pouring a centrifuged clear liquid back into the rotary screen, and performing enzymatic hydrolysis cyclically for 2-3 times; and

S24. uniformly stirring the corn slurry having undergone the enzymatic hydrolysis and filtering, collecting a filtrate, and into the filtrate, adding an antioxidant of 0.5%-1.5% of a weight of the filtrate; and

S3. extraction and post-treatment:

S31. sonicating the filtrate to obtain a liquid, and adding any one or more of an activator, a protective agent and a stabilizer into the liquid; and
S32. concentrating and drying: putting the filtrate in a low-temperature vacuum concentrator, and performing evaporation at 22-25 °C for 8-15 hours, so as to obtain a corn superoxide dismutase multienzyme liquid; and uniformly mixing the corn superoxide dismutase multienzyme liquid with silica accounting for 1% of a total weight, placing the mixture into a low-temperature vacuum drying device, and freeze-drying the same, so as to obtain a corn superoxide dismutase multienzyme powder.

Further, an axis of the drum of the rotary screen is horizontally provided, a diameter of meshes of the drum is smaller than a particle size of pulverized corn, an opening at one end is configured for charging, and the other end is provided with a discharge port.

Further, an amount of the corn slurry added in the S21 is 40%-60% of a volume of the rotary screen; the enzymatic hydrolysis mixed liquid includes an enzyme complex and a surfactant, where the enzyme complex is a mixture of pectinase, cellulase, hemicellulase and protease, an amount of the enzyme complex added is 0.5%-2% of a mass of the corn slurry, the surfactant is sodium dodecyl sulfonate, and added at 0.5%-0.8% of the mass of the corn slurry; an enzymatic hydrolysis temperature is 45-55 °C, a pH value is 7.0-8.0, and an enzymatic hydrolysis duration is 40-80 minutes.

Further, multiple sets of rotating blades are further installed in the rotary screen; and an object dynamic model is used to simulate object flow during stirring, so as to ensure thorough uniform stirring; and specifically, a plurality of blades are installed in the rotary screen, initially, a helical blade shape is selected, with a blade width being 10%-20% of a diameter of the rotary screen, and a blade length being slightly less than half of a length of the rotary screen, the blades are arranged in a staggered manner, and an angle of the blades is 30-60°.

Further, a transmittance sensor is further installed on a tank body storing the enzymatic hydrolysis mixed liquid, and a mapping relationship model of transmittance with starch concentration is established, specifically:

quantifying the mapping relationship model of relevant transmittance with starch concentration based on historical data and experimental calibration, and determining analysis and fitting of relevant quantitative data by actual situations;

photographing a state of the material in the rotary screen by a camera below the rotary screen, performing feature extraction and identification, and calculating an immersion ratio of starch granules in the mixed liquid so as to infer enzymatic hydrolysis progress;
performing a grayscale histogram analysis on a collected original image;
subsequently performing a filtration denoising pretreatment operation, so as to extract features of the starch granules;
applying morphological operations to further clean the image, remove noises, connect adjacent starch-granule regions and extract outline edges of the starch granules;
identifying all starch-granule regions in the image by a connected component labeling algorithm, and calculating an area and shape features of each region;
classifying the extracted features, and distinguishing the starch granules from other impurities;
calculating the number of contact pixels between the starch-granule region and a mixed-liquid background region to approximate an immersion ratio;
setting a threshold based on acquired data of total starch amount, carrying out step S221, and when the starch concentration exceeds the set threshold, starting a centrifugal separation device; and
after step S221 is ended, carrying out step S231: performing the above operations iteratively until extraction and separation of the superoxide dismutase is completed.

Further, after the centrifugal separation device is started in step S221, a solid precipitate and a supernatant are separated, where the solid precipitate is primarily undissolved starch granules.

Further, the solid precipitate obtained after centrifugation is collected, dried and then weighed using a precision balance, and a mass of the precipitate is recorded, specifically including:

S2211. comparing an initial amount of corn flour and a starch content, and calculating an amount of starch to be obtained theoretically;
S2212. calculating a degree of completion of the enzymatic hydrolysis progress by comparing the starch amount in actual precipitate with the theoretical starch amount, so as to obtain a percentage of the degree of completion of the enzymatic hydrolysis progress,
where the degree of completion of the enzymatic hydrolysis progress is $CR = \frac{Ma}{Ms} * 100\%$,
where Ma is an actual starch amount measured by weighing after drying; and
S2213. analyzing the above steps, so as to evaluate influence of enzymatic hydrolysis conditions such as temperature, pH value, and enzyme dosage on the degree of completion of the progress.

Further, after recording and analyzing the mass of the precipitate, a sensor and a data logging system are further provided, so as to obtain a continuous dynamic data set, including variations in various parameters during the enzymatic hydrolysis, and the enzymatic hydrolysis progress and overall temporal data for the same batch of material are monitored and recorded in real time.

Further, prior to enzymatic hydrolysis of each batch of material, data of first three batches are collected, a mean value is calculated, and a baseline standard for an enzymatic hydrolysis operation is established, where a baseline value formula of the enzymatic hydrolysis operation is as follows:

$$\text{Baseline} = \frac{1}{n}\sum\nolimits_{i=1}^{n} (\text{Data}_{\text{batch\_i}})$$

where n represents the number of data points for which the mean value is to be calculated, and in this implementation scenario, it refers to the number of data of the first three batches, $\text{Data}_{\text{batch\_i}}$ represents a data value of an i-th batch, and $\frac{1}{n}\sum_{i=1}^{n}$ represents a sum of all data values from a first batch to an n-th batch, the formula means that the mean value of the data is calculated by dividing a sum of the data values of the first three batches by the number of batches, and the mean value is taken as the baseline value.

Further, based on real-time progress of starch filtration in the rotary screen, a rotational speed of the rotary screen and a stirring interval of the mixed liquid are dynamically adjusted, so as to achieve an optimal enzymatic hydrolysis effect;

distribution and concentration of starch in the mixed liquid are analyzed by combining a single filtration amount and a total cumulative amount of starch, and a liquid discharge and flushing frequency of a centrifuge is automatically adjusted;

when the stirring is ceased, the camera captures an image of an upper part of the mixed liquid in the rotary screen, and the image is converted into a grayscale image for analyzing distribution and concentration of starch granules;

grayscale value variations in different regions of the grayscale image are analyzed through an image processing algorithm, and real-time progress and estimated amount of starch filtration are obtained by determining progress and mass of the starch filtration;

effectiveness of current enzymatic hydrolysis parameters is evaluated by comprehensively analyzing data such as the rotational speed of the rotary screen, stirring interval, centrifuging frequency, and image identification result; and

based on evaluation results, enzymatic hydrolysis control parameters are adjusted.

One or more technical solutions provided in the embodiments of the present invention at least have the following technical effects or advantages.

Firstly, by configuring the enzymatic hydrolysis process on the rotary screen, and immersing the lower half of the drum in the enzymatic hydrolysis mixed liquid, the material is immersed in the enzymatic hydrolysis mixed liquid all the time; at the same time, the diameter of meshes of the drum is smaller than the particle size of the pulverized corn, and the starch granules can pass through the meshes, thus facilitating filtration and removal of starch. By providing the rotary screen, effective filtration and removal of the starch granules is achieved, and at the same time, it ensures sufficient contact and enzymatic hydrolysis of corn cell walls by the enzymatic hydrolysis mixed liquid. Through dynamic rolling of the rotary screen and cyclic updating of the enzymatic hydrolysis mixed liquid, the efficiency of enzymatic hydrolysis is improved, and the interference of starch on the enzymatic hydrolysis process is reduced, thus effectively solving the problem of viscosity caused by the high starch content when using corn as the raw material for producing SOD, significantly improving the efficiency of enzymatic hydrolysis, effectively protecting the activity of SOD, and rendering stable and reliable product quality; at the same time, the technical solution facilitates large-scale production, reduces production costs, and improves economic efficiency.

Secondly, by providing a stirring system, uniform dispersion of the starch granules in the enzymatic hydrolysis mixed liquid is ensured, thus avoiding the problem of viscosity caused by excessive local concentration, and improving contact efficiency of enzyme with corn cells. The transmittance data of the real-time transmittance monitoring system timely reflect release and dispersion situations of starch in the mixed liquid, thus providing a basis for adjusting the stirring parameters. Through the image analysis and calculation of starch immersion amount, non-intrusive real-time enzymatic hydrolysis progress monitoring is achieved, thus providing accurate data support for process adjustment. By controlling the total starch amount in the mixed liquid and the centrifugal separation process, excessive starch in the mixed liquid is effectively removed, thus reducing the viscosity while recovering starch resources, thereby improving economic efficiency of the whole process.

Thirdly, by further optimizing the starch amount analysis step after the centrifugal separation, quantitative evaluation of the degree of completion of the enzymatic hydrolysis progress is realized. By comparing a difference between the actual starch amount and the theoretical starch amount, efficiency of enzymatic hydrolysis reaction can be visually reflected, and key data support is provided for process optimization, thus improving controllability and predictability of a production process, thereby improving product quality and production efficiency. Through measures of stirring system optimization, real-time transmittance monitoring, image analysis and starch immersion amount calculation, controlled total starch amount in the mixed liquid and centrifugal separation, the efficiency of enzymatic hydrolysis of the process of extracting SOD from the corn raw material is significantly improved. Sufficient contact of enzyme with core cells is ensured, the enzymatic hydrolysis time is shortened, and the yield of SOD is improved. Through the real-time monitoring and data analysis, precise control over the enzymatic digestion progress is achieved, and human intervention errors are reduced. Starch resources are effectively recycled, thus reducing waste generation, and elevating economic efficiency and environmental protection of the process.

Fourthly, by introducing advanced technical means such as data continuous monitoring, baseline establishment, active control, and image identification, intelligent management and optimization of an enzymatic hydrolysis process are achieved. Through real-time data collection and analysis, in conjunction with an image identification technology, the starch filtration progress can be accurately determined, and the enzymatic hydrolysis control parameters are accordingly dynamically adjusted. This closed-loop feedback mechanism not only improves the efficiency of enzymatic hydrolysis, but also ensures stability and controllability of the production process, can significantly shorten the enzymatic hydrolysis time, improve the product activity, and reduce the production cost and energy consumption at the same time.

**BRIEF DESCRIPTION OF DRAWINGS**

FIG. 1 is a schematic diagram of a rotary screen in Example 1 of the present invention; and
FIG. 2 is a flowchart of photographing a state of a material in the rotary screen, performing feature extraction and identification, calculating an immersion ratio of starch granules in a mixed liquid and inferring enzymatic hydrolysis progress.

**DETAILED DESCRIPTION OF EMBODIMENTS**

Unless otherwise defined, all technical and scientific terms used herein have the same meanings as those generally understood by those skilled in the art to which the present invention belongs; the terms used in the description of the present invention are merely for the purpose of describing embodiments, but are not intended to limit the present invention; and the terms "and/or" used herein includes any and all combinations of one or more relevant items listed.

Example 1: A preparation method for a corn superoxide dismutase, specifically including following steps:

S1. pre-treatment

S11. wall breaking of yeast fermentation broth: inoculating yeast into a medium, performing fermentation under conditions of 28-32 °C and pH of 4.5-5.5 for 48-72 hours, and then adding snailase for reacting for 1-2 hours, so as to obtain a wall-broken yeast fermentation broth, where an amount of inoculated yeast was 5%-10% of a volume of the medium, and a ratio of snailase added was 50-100 mg per liter of the fermentation broth;
S12. material preparation: preparing a suitable amount of raw material corn, and removing impurities from the corn for later use;
S13. germination: placing the corn into a fully automated apparatus for seed cleaning, steeping, and germinating, cleaning and steeping for 8-12 hours, germinating at 25-30 °C for 48-60 hours until a sprout length was 3-5 mm, where a steeping liquid contained the wall-broken yeast fermentation broth, with an amount of the wall-broken yeast fermentation broth being 1%-5% of a weight of corn, and further included 5-10 mg/L iron, 0.1-0.5 mg/L selenium, 1-2 mg/L copper, 5-10 mg/L manganese, and 5-10 mg/L zinc; and
S14. pulverizing: pulverizing sprouted corn to 80-120 mesh, into the pulverized sprouted corn, adding a pH=7.8, 0.05 mol/L phosphate-buffered saline (PBS) at a ratio of 1:2-4, and mixing uniformly and grinding the mixture into a corn slurry, followed by ultrasonic cell disruption;

S2. enzymatic hydrolysis

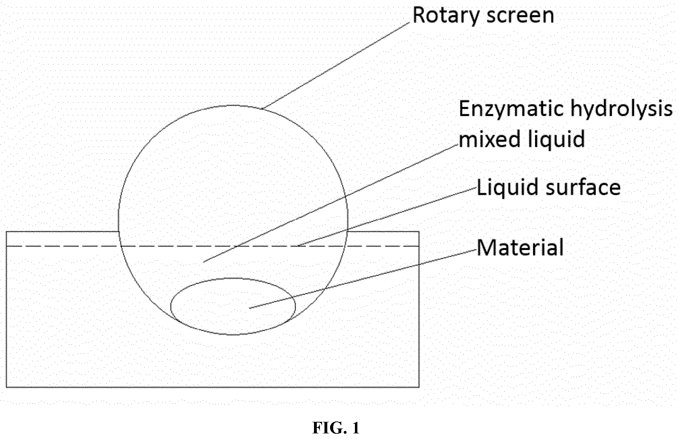

as shown in FIG. 1, a rotary screen was provided and mounted, an axis of a drum was horizontally provided, an opening at one end was configured for charging, and the other end was provided with a discharge port, where a diameter of meshes of the drum was less than a particle size of pulverized corn;
S21. transferring the corn slurry having undergone ultrasonic disruption in S14 into the rotary screen, and immersing a lower half of the drum into an enzymatic hydrolysis mixed liquid; starting the rotary screen, so as to enable the corn material to tumble inside the drum while remaining immersed in the enzymatic hydrolysis mixed liquid, where an amount of the corn slurry added was 40%-60% of a volume of the rotary screen; the enzymatic hydrolysis mixed liquid included an enzyme complex and a surfactant, where the enzyme complex was a mixture of pectinase, cellulase, hemicellulase and protease, an amount of the enzyme complex added was 0.5%-2% of a mass of the corn slurry, the surfactant was sodium dodecyl sulfonate, and added at 0.5%-0.8% of the mass of the corn slurry; an enzymatic hydrolysis temperature was 45-55 °C, a pH value was 7.0-8.0, and an enzymatic hydrolysis duration was 40-80 minutes;

S22. extracting the enzymatic hydrolysis mixed liquid every 10-15 minutes to undergo centrifugal separation, so as to remove suspended starch granules and other impurities;

S23. pouring the centrifuged clear liquid back into the rotary screen, and performing enzymatic hydrolysis cyclically for 2-3 times; and

S24. uniformly stirring the corn slurry having undergone the enzymatic hydrolysis and filtering, collecting a filtrate, and into the filtrate, adding an antioxidant of 0.5%-1.5% of a weight of the filtrate; and

S3. extraction and post-treatment

S31. sonicating the filtrate to obtain a liquid, and adding any one or more of an activator, a protective agent and a stabilizer into the liquid, where a ultrasonic frequency was 20-30 kHz, power was 10-20 W, ultrasonic operation was performed for 15-20 s, followed by 5-10 s of interval, a total ultrasonication duration was 10-20 min, and a temperature in an extraction process was 30-60 °C; the activator was manganese chloride and ferrous chloride; the protective agent was a protein disulfide bond protective agent; and the stabilizer was trehalose or burdock oligosaccharide; and

S32. concentrating and drying: putting the filtrate in a low-temperature vacuum concentrator, and performing evaporation at 22-25 °C for 8-15 hours, so as to obtain a corn superoxide dismutase multienzyme liquid; and

uniformly mixing the corn superoxide dismutase multienzyme liquid with silica accounting for 1% of a total weight, placing the mixture into the low-temperature vacuum drying device, and freeze-drying the same, so as to obtain a corn superoxide dismutase multienzyme powder.

The technical solution in above example of the present invention at least has the following technical effects or advantages.

A starch content in corn is about 48%-73%, and when corn is used as a raw material for producing SOD, especially for industrial-scale production, after starch granules are released during enzymatic hydrolysis, liquid is viscous during the enzymatic hydrolysis, hindering contact of enzyme with cell walls, resulting in uneven distribution of enzyme, reducing efficiency of enzymatic hydrolysis, and increasing release of impurities, thus affecting activity and extraction amount of SOD.

By configuring the enzymatic hydrolysis process on the rotary screen, and immersing the lower half of the drum in the enzymatic hydrolysis mixed liquid, the material is immersed in the enzymatic hydrolysis mixed liquid all the time; at the same time, the diameter of meshes of the drum is smaller than the particle size of the pulverized corn, and the starch granules can pass through the meshes, thus facilitating filtration and removal of starch. By providing the rotary screen, effective filtration and removal of the starch granules is achieved, and at the same time, it ensures sufficient contact and enzymatic hydrolysis of corn cell walls by the enzymatic hydrolysis mixed liquid. Through dynamic rolling of the rotary screen and cyclic updating of the enzymatic hydrolysis mixed liquid, the efficiency of enzymatic hydrolysis is improved, and the interference of starch on the enzymatic hydrolysis process is reduced, thus effectively solving the problem of viscosity caused by the high starch content when using corn as the raw material for producing SOD, and significantly improving the efficiency of enzymatic hydrolysis, effectively protecting the activity of SOD, and rendering stable and reliable product quality; at the same time, the technical solution facilitates large-scale production, reduces production costs, and improves economic efficiency.

The preparation of superoxide dismutase through the solution of the present example can effectively extract SOD from corn, and improve extraction efficiency. Adjustment of process parameters in the present example can protect the activity of SOD, reduce loss in an extraction process, and improve purity and activity of the product. By optimizing steps of pre-treatment, enzymatic hydrolysis, extraction and post-treatment, material consumption and energy consumption in the production process are reduced, thereby reducing production costs. By optimizing the extraction and purification processes, and high purity and high activity of a finished SOD product are ensured, thus meeting requirements for the SOD product in fields of food, cosmetics, health care products, agriculture and so on.

Example 2: in the above Example 1, through the dynamic rolling of the rotary screen and cyclic updating of the enzymatic hydrolysis mixed liquid, the efficiency of enzymatic hydrolysis is improved, the interference of starch on the enzymatic hydrolysis process is reduced, the viscosity problem caused by the high starch content when using corn as the raw material for producing SOD is effectively solved, the efficiency of enzymatic hydrolysis is significantly improved, and the activity of SOD is effectively protected. In order to further improve the efficiency of enzymatic hydrolysis, and improve the activity of SOD, further improvement was made on the basis of step S2 in Example 1.

S211. further installing multiple sets of rotating blades in the rotary screen; and using an object dynamic model to simulate object flow during stirring, thus ensuring thorough uniform stirring.

Specifically, a plurality of blades were installed in the rotary screen. Initially, a helical blade shape was selected, with a blade width being 10%-20% of a diameter of the rotary screen; and a blade length being slightly less than half of a length of the rotary screen, thus ensuring coverage of a primary stirring region without increasing excessive resistance. The blades were arranged in a staggered manner, and an angle of the blades was 30-60°. Fluid dynamics was calculated by CFD, geometric dimensions of the rotary screen, design parameters of the blades, physical properties such as density and viscosity of the material, stirring speed and other conditions were input, to simulate a flow track and a mixing effect of the material in the rotary screen. Based on simulation results, the number, shape, arrangement manner and rotational speed of the blades and other parameters were adjusted, so as to achieve a thorough uniform stirring effect, and the optimized parameters were used for designing the rotary screen in practical application production.

Moreover, a transmittance sensor was installed on a tank body storing the enzymatic hydrolysis mixed liquid, and a mapping relationship model of the transmittance with starch concentration was established.

Specifically:
the mapping relationship model of relevant transmittance with starch concentration was quantified based on historical data and experimental calibration, and analysis and fitting of relevant quantitative data were determined by actual situations, which will not be repeated herein.

For example, mixture samples at different starch concentrations were collected through experiment, and they were measured for transmittance values. Through data analysis and fitting, a quadratic polynomial regression model was obtained, where the model can be expressed as:

$$C = \alpha T^2 + \beta T + \gamma$$

In the above, C is starch concentration after mapping, expressed in g/L, T is the transmittance value expressed in percentage, and $\alpha$, $\beta$ and $\gamma$ are coefficients obtained through data fitting. In this context, $\alpha$=0.001, $\beta$=0.15, and $\gamma$=5.0 were obtained, and then the mapping relationship model can be quantified as:

$$C = 0.001T^2 + 0.15T + 5.0$$

It is assumed that a hypothetical data set was constructed, 80% transmittance is corresponding to 12.5 g/L starch concentration, encompassing starch concentration values corresponding to different transmittance values therein. These data were measured through experiment, and they are supposed to be error-free or have negligible error. Moreover, the transmittance obtained through measurement was 80%, and it was put into the model to calculate the starch concentration, so that the starch concentration after the mapping was C=12.6 g/L, and the corresponding starch concentration 12.6 g/L was close to an actually measured value 12.5 g/L, indicating excellent model fitting.

S212, photographing a state of the material in the rotary screen by a camera below the rotary screen, performing feature extraction and identification, and calculating an immersion ratio of starch granules in the mixed liquid so as to infer enzymatic hydrolysis progress.

Herein, in the mixing process, when stirring was stopped, a 16320×9200 high-resolution camera was used to take an image of an upper part of the mixed liquid in the rotary screen. Within 10 seconds after the stirring was stopped, a grayscale image of the upper part of the mixed liquid in the rotary screen was taken.

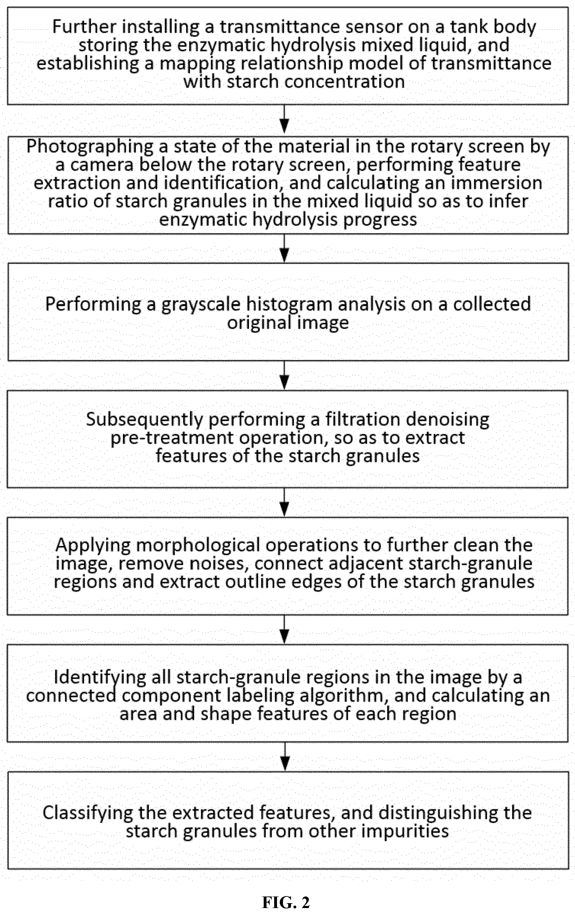

As shown in FIG. 2, the step of photographing a state of the material in the rotary screen, performing feature extraction and identification, calculating an immersion ratio of starch granules in the mixed liquid and inferring enzymatic hydrolysis progress includes following steps.

In some examples, the step of photographing a state of the material in the rotary screen, performing feature extraction and identification, calculating an immersion ratio of starch granules in the mixed liquid and inferring enzymatic hydrolysis progress specifically included:
performing a grayscale histogram analysis on a collected original image.

Specifically, the collected original color image was converted into a grayscale image. The histogram analysis was performed on the grayscale image, that is, the number of pixels at different grayscale levels in the image was counted. The histogram is a two-dimensional graph, in which X-axis represents the grayscale level, and Y-axis represents the number of pixels at the grayscale level. Due to the presence of starch granules, the grayscale histogram shows a remarkable peak near 255 in a high-grayscale-value region. As the starch granules are generally brighter than background, they show higher grayscale values in the grayscale image. The histogram was observed to find whether a significant peak appeared in a higher-grayscale-value region 180-255. This peak should be significantly higher than the number of pixels at the other grayscale levels, and is relatively narrow in width, indicating a high probability that pixels corresponding to these grayscale values are starch granules. Based on the grayscale histogram, a valley value between a starch granule peak and a background peak in the histogram was chosen as a threshold to distinguish a starch-rich region and a mixed-liquid background (assuming that the histogram shows a remarkable starch granule peak around a grayscale value 200, while the background peak is primarily at the grayscale value below 100. Then threshold 150 was chosen to distinguish the starch-rich region and the background).

Subsequently, a filtration denoising pre-treatment operation was carried out, so as to extract features of the starch granules.

Specifically, color segmentation was performed first for the feature extraction of the image, as the starch granules and the mixed-liquid background have differences in color, the image was segmented into K clusters based on color feature RGB values of the pixel points, and each cluster is assigned a unique label. After distribution is completed, each pixel point owned a cluster label, indicating the cluster to which it belongs, a color feature of each cluster were analyzed, and the cluster closest to the color feature of the starch granules is found, thus separating the starch granules from the background.

Morphological operations were applied to further clean the image, remove noises, connect adjacent starch-granule regions and extract outline edges of the starch granules.

Herein, the applied morphology included erosion, dilation, opening operation and closing operation. The erosion operation can remove some small noise points at edges of the starch granules and make edges thereof shrink inwards; a dilation operation can fill some small voids in the starch granules and make edges thereof dilate outwards; the opening operation can further clean the image, while maintaining an overall shape of the starch granules; and the closing operation is mainly used for connecting adjacent starch-granule regions, so as to form them into a complete connected region.

Specifically, a circle was chosen as a basic structural element, a size of the circle should be adjusted according to an actual size of the starch granules. The structural element was slid on the image, and for each position, if all pixels within the structural element belonged to the starch granule cluster, a pixel in a central position of the structural element was retained; otherwise, the pixel was set as background. The same structural element as the erosion operation was used, the structural element was slid on the image, and for each position, if any pixel in the structural element belonged to the starch granule cluster, a pixel in a central position of the structural element was set as a starch granule, where the erosion operation enabled a target region range to "become smaller", and removed small noise points at edges. The opening operation was a process of first erosion and then dilation, where an erosion operation was first performed on the image so as to remove small noise points and burrs; the result after the erosion was then subjected to the dilation operation to recover eroded portions, but to maintain the effect of removing noises and burrs. The closing operation was a process of first dilation and then erosion, where the dilation operation was performed on the image to fill in small voids inside the starch granules; subsequently, the result after the dilation was subjected to the erosion operation to remove extra edges that may be generated during the dilation, and adjacent granule regions were connected to form a complete connected region. A single erosion or dilation operation may not achieve a desired cleaning effect. In this case, it may be considered to perform multiple iteration operations on the image, that is, the erosion, dilation, opening operation or closing operation was repeatedly executed until requirements were met. An outline edge extraction adopted a Canny edge detection algorithm, and for relevant operations, reference is made to the prior art, which is not repeated herein.

All starch-granule regions in the image were identified by a connected component labeling algorithm, and an area and shape features of each region were calculated.

Herein, in the connected component analysis, a Two-Pass algorithm suitable for binary image processing was selected.

Specifically, an output image with the same size as an input image but of a data type of integer type (for storing label) was created. Typically, a label of background pixels is set to be 0, and an initial label of foreground (i.e., starch granule) pixels was set to be a provisional value (which is subsequently replaced with a unique label). Starting from a top left corner of the image, the whole image was scanned pixel by pixel. For each foreground pixel (i.e., starch granule pixel), pixels within 4-connected or 8-connected neighborhood thereof were checked. If neighborhood pixels included a foreground pixel not assigned a label, it was classified as the same connected component as the current pixel, and they were assigned the same provisional label. If the neighborhood pixels included foreground pixels assigned different temporal labels, these different labels were recorded for subsequent processing. At the end of a first pass scanning, an equivalence relationship between a plurality of provisional labels was recorded, i.e., which provisional labels represented the same connected component.

Each connected component was assigned a unique identifier based on the equivalence relationship recorded in the first pass scanning. This is typically accomplished by traversing all provisional labels and replacing them with the minimal label within an equivalence class thereof. Upon completing a second pass scanning, a labeled image was output, where each connected component was assigned a unique identifier. An area of each connected component was calculated by counting the number of pixels therein. A variety of shape features can be calculated, such as perimeter (calculated by traversing boundary pixels of the connected component), circularity (estimated through an area-perimeter relationship), aspect ratio (calculated from length and width of the minimum bounding rectangle), etc.

The extracted features were classified, and the starch granules were distinguished from other impurities.

Specifically, the shape features such as the area, perimeter, and circularity of each component were acquired as input features from the connected component analysis. A group of image samples of a known class were collected, including the starch granules and other impurities; and the connected component analysis was performed on each sample, and corresponding features were extracted. As dimensions and ranges of different features may be different, it was necessary to standardize the features, so as to ensure fair contribution of each feature to a classifier. The standardized feature data set was used to train an SVM classifier. During the training, the SVM found a hyperplane that can maximize a margin between different classes. The connected component analysis was performed on new image samples, and corresponding features were extracted. Then, the same normalization method was used to process the features. The normalized features were input into the trained SVM classifier, which output probability or a decision value of each sample belonging to starch granules or other impurities. The class of each sample can be determined based on the output of the classifier. With regard to the probability output, a threshold can be set to determine a final class. For the decision value output, the class can be determined directly according to a sign (positive or negative). In the above scenario of distinguishing the starch granules and other impurities, the normalization processing specifically refers to a series of transformation operations applied to the shape features such as area, perimeter, and circularity of each region extracted from the connected component analysis, and for relevant specific steps, reference can be made to the prior art, and they are not repeated herein.

S213, calculating the number of contact pixels between the starch-granule region and the mixed-liquid background region to approximate an immersion ratio.

Specifically, each starch-granule region was traversed, and the number of edge pixels in contact with the mixed-liquid background was counted. The number of pixels was converted into an actual surface area based on a pixel size and an image resolution. The total number of pixels in each starch-granule region was calculated, and similarly converted into an actual surface area.

Herein, the immersion ratio is defined as a ratio of a surface area of the starch granules in contact with the mixed liquid to a total surface area of the starch granules, that is, immersion ratio = surface area corresponding to the number of contact pixels / total surface area of the starch granules. Before executing this step, it was necessary to take images of the starch mixed liquid at different enzymatic hydrolysis time points in a laboratory by controlling experimental condition variables such as enzyme concentration, temperature, and pH value, the above processing procedure was conducted on these images, to calculate the immersion ratio of the starch granules at each time point. Based on experimental data, a relationship model between the immersion ratio of the starch granules and enzymatic hydrolysis time was established, and the established relationship model was applied. In practical production process, images of the state of the material in the drum are acquired in real time. The enzymatic hydrolysis progress is inferred based on the currently calculated immersion ratio of the starch granules.

S221, setting a threshold based on the acquired data of total starch amount, and starting a centrifugal separation device when the starch concentration exceeded the set threshold.

Specifically, according to actual situations, it is set that when a concentration reached x% at a first startup of device, a first extraction and separation of superoxide dismutase is started, a second start threshold is set to be y% (y>x), and a third one is set to be z% (z>y), and so on until the superoxide dismutase is finally extracted and separated. That is, when the data of the total starch amount acquired in real time exceeds the threshold, it is considered that the starch concentration in the mixed liquid has reached a standard requiring further purification and processing.

For example, an initial concentration of superoxide dismutase is 80%, and target purity is 99.9%. A threshold of a first purification is set such that when the concentration reaches 85%, the device is started. The device is started, and a purification process is started. The purification is ceased when the concentration approaches but does not exceed 90%, assuming a concentration after the purification being 88%. A threshold of a second purification is set such that when the concentration reaches 90%, the device is started. The device is started, and the purification is continued. The purification is ceased when the concentration approaches but does not exceed 95%, assuming a concentration after the purification being 92.5%. A threshold of a third purification is set such that when the concentration reaches 95%, the device is started. The device is started, and the last purification is performed. The purification is stopped when the concentration approaches the target purity 99.9%, assuming a concentration after the final purification being 99.8%.

S231, performing the above operations iteratively until the extraction and separation of the superoxide dismutase was completed.

The technical solution in the above example of the present invention at least has the following technical effects or advantages.

By providing a stirring system, uniform dispersion of the starch granules in the enzymatic hydrolysis mixed liquid is ensured, thus avoiding the problem of viscosity caused by excessive local concentration, and improving contact efficiency of enzyme with corn cells. The transmittance data of the real-time transmittance monitoring system timely reflect release and dispersion situations of starch in the mixed liquid, thus providing a basis for adjusting the stirring parameters. Through the image analysis and calculation of starch immersion amount, non-intrusive real-time enzymatic hydrolysis progress monitoring is achieved, thus providing accurate data support for process adjustment. By controlling the total starch amount in the mixed liquid and the centrifugal separation process, excessive starch in the mixed liquid is effectively removed, thus reducing the viscosity while recovering starch resources, thereby improving economic efficiency of the whole process.

Example 3: in the above Example 2, by providing the stirring system, real-time transmittance monitoring, image analysis, starch immersion amount calculation, and controlling the total starch amount in the mixed liquid and centrifugal separation, efficiency of enzymatic hydrolysis in a process of extracting SOD from the corn raw material is significantly improved. In order to further improve the efficiency of enzymatic hydrolysis, step S221 was further improved on the basis of Example 2.

The mixture having undergone the enzymatic hydrolysis was subjected to centrifugal separation using a high-speed centrifuge, so as to separate a solid precipitate and a supernatant.

Herein, the solid precipitate was primarily undissolved starch granules.

The solid precipitate obtained after centrifugation was further collected, dried and then weighed using a precision balance, and a mass of the precipitate was recorded.

Herein, accuracy of the balance can reach at least 0.01 gram, i.e., hundredth gram.

Specifically:

S2211. comparing an initial amount of corn flour and a starch content, and calculating an amount of starch to be obtained theoretically.

Specifically, a mass of the separated corn starch granule precipitate was recorded as Mn, a known percentage of corn starch content was P, and a theoretical starch mass Ms=Mn*P generally can be obtained from product description or previous analysis, thus providing a reference standard for evaluating a degree of completion of enzymatic hydrolysis progress.

S2212. calculating the degree of completion of the enzymatic hydrolysis progress by comparing the starch amount in the actual precipitate with the theoretical starch amount, so as to obtain a percentage of the degree of completion of the enzymatic hydrolysis progress.

Herein, the degree of completion of the enzymatic hydrolysis progress is $CR = \dfrac{Ma}{Ms} * 100\%$, where Ma is the actual starch amount measured by weighing after drying.

S2213. analyzing the above steps, so as to evaluate influence of the enzymatic hydrolysis conditions such as temperature, pH value, and enzyme dosage on the degree of completion of the progress.

The technical solution in the above example of the present invention at least has the following technical effects or advantages.

By further optimizing the starch amount analysis step after the centrifugal separation, quantitative evaluation of the degree of completion of the enzymatic hydrolysis progress is realized. By comparing a difference between the actual starch amount and the theoretical starch amount, efficiency of enzymatic hydrolysis reaction can be visually reflected, and key data support is provided for process optimization, thus improving controllability and predictability of a production process, thereby improving product quality and production efficiency.

Through measures of stirring system optimization, real-time transmittance monitoring, image analysis and starch immersion amount calculation, controlled total starch amount in the mixed liquid and centrifugal separation, the efficiency of enzymatic hydrolysis of the process of extracting SOD from the corn raw material is significantly improved.

Sufficient contact of enzyme with core cells is ensured, the enzymatic hydrolysis time is shortened, and the yield of SOD is improved. Through the real-time monitoring and data analysis, precise control over the enzymatic digestion progress is achieved, and human intervention errors are reduced. Starch resources are effectively recycled, thus reducing waste generation, and elevating economic efficiency and environmental protection of the process.

Example 4: by further optimizing centrifugal separation and starch amount analysis in the above Example 3, quantitative evaluation of the degree of completion of the enzymatic hydrolysis progress is realized, and controllability and predictability of the production process are improved, thus improving the product quality and production efficiency. In order to further improve the efficiency of enzymatic hydrolysis, further improvement was made on the basis of Example 3.

A sensor and a data logging system were provided, so as to obtain a continuous dynamic data set, including variations in various parameters during the enzymatic hydrolysis. The enzymatic hydrolysis progress and overall temporal data for the same batch of material were monitored and recorded in real time.

Data of first three batches were collected, a mean value was calculated, and a baseline standard for an enzymatic hydrolysis operation was established.

Herein, a baseline value formula of the enzymatic hydrolysis operation is as follows:

$$Baseline = \frac{1}{n}\sum_{i=1}^{n} (Data_{batch\_i})$$

where n represents the number of data points for which the mean value is to be calculated, and in this context, it refers to the number of data of the first three batches (i.e., n=3, but the formula is written in a more general form and can be applied to any number of batches), $Data_{batch\_i}$ represents a data value of an i-th batch, and $\dfrac{1}{n}\sum_{i=1}^{n}$

represents a sum of all data values from the first batch to the n-th batch. The formula means that the mean value of the data is calculated by dividing a sum of the data values of the first three batches (or any specified number of batches) by the number of batches, and the mean value is taken as the baseline value.

Based on real-time progress of starch filtration in the rotary screen, a rotational speed of the rotary screen and a stirring interval of the mixed liquid were dynamically adjusted, so as to achieve an optimal enzymatic hydrolysis effect.

Specifically, according to factors such as viscosity, moisture content, and gelatinization temperature of the starch raw material, the rotational speed range of the rotary screen can be initially set at 200-600 revolutions per minute. Based on the real-time progress of starch filtration, the rotational speed of the rotary screen was dynamically adjusted, and for subsequent rotational speed adjustment, each adjustment did not exceed 50 revolutions per minute, and the adjustment was performed every 10-30 minutes. Initial stirring lasted no less than 5 minutes, and in an initial enzymatic hydrolysis stage, a stirring interval can be appropriately shortened to every 5-10 minutes, so as to promote sufficient contact and reaction between enzyme and starch. In a mid-stage of enzymatic hydrolysis, the stirring interval can be appropriately extended to 15-20 minutes as the reaction progressed, so as to reduce energy consumption and equipment wear. In a later stage of enzyme hydrolysis, when approaching a reaction endpoint, the stirring interval can be extended or the stirring was ceased, so as to avoid influence of over-stirring on starch structure.

Distribution and concentration of starch in the mixed liquid were analyzed by combining a single filtration amount and a total cumulative amount of starch, and a liquid discharge and flushing frequency of the centrifuge was automatically adjusted.

Specifically, a filtration amount of starch in each centrifugal process was monitored in real time through a weighing device, and a data system was established to record a cumulative amount. A volume of the mixed liquid was estimated from charge and discharge volumes of the centrifuge, and further the starch concentration was calculated (C=M/V, where C is the concentration, M is the starch mass, and V is the mixed liquid volume). The concentration was calculated every 10 minutes, synchronized with filtration amount monitoring. In an initial stage, samples were collected every 0.5 hours at different locations for concentration analysis, with an upper limit set to be 12% and a lower limit set to be 8%. When the concentration exceeded the upper limit, a liquid discharge and flushing frequency of the centrifuge was automatically increased by 20% until the concentration fell below the upper limit; when the concentration was lower than the lower limit, the frequency was automatically reduced by 10% until the concentration rose back to above the lower limit. An initial flushing frequency was once every 30 minutes. For example, if the concentration was monitored to consistently rise to 12.5%, the flushing frequency was automatically adjusted to once every 24 minutes. After adjustment, when the concentration fell to 10%, the current frequency was maintained or fine-tuned as needed. If the concentration remained below 8%, the frequency was automatically adjusted to once every 33 minutes, and so on.

When the stirring was ceased, the camera captured an image of an upper part of the mixed liquid in the rotary screen, and the image was converted into a grayscale image for analyzing the distribution and concentration of starch granules.

Herein, the captured color image was converted into a grayscale image which converted a color value of each pixel to a luminance value between 0 and 255.

Grayscale value variations in different regions of the grayscale image were analyzed through an image processing algorithm, and real-time progress and estimated amount of starch filtration were obtained by determining progress and mass of the starch filtration.

Specifically, in the starch filtration process, the grayscale image was first divided into a plurality of ROI regions, and grayscale values of pixels in each region were extracted. These grayscale values directly reflect presence and concentration of starch granules. An average concentration of starch granules and distribution thereof can be learned by counting the average grayscale value and the grayscale value distribution of each ROI. Upon continuous filtration, starch granules in the mixed liquid were reduced, resulting in a reduced grayscale value. By comparing the grayscale images and grayscale value variations at different time points, trend and speed the starch filtration can be analyzed. Specifically, a continuous decrease in the grayscale value indicates that the filtration is ongoing and the effect is significant; and if the grayscale value variation is stable or ceases to decrease, it means that the filtration has reached a steady state or requires an adjustment operation. In order to estimate a filtration amount of starch, a mathematical model was established based on experimental data and empirical data, and the grayscale value variations were converted into the filtration amount of starch. This typically involves establishment of a linear or non-linear relationship between the grayscale values and the starch concentrations. By continuously capturing and analyzing images, progress information of starch filtration was acquired in real time, and a filtration amount of residual starch was estimated based on the current filtration progress and a grayscale value variation trend. This estimation process requires appropriate prediction and calibration with reference to historical data and empirical data.

Effectiveness of current enzymatic hydrolysis parameters was evaluated by comprehensively analyzing data such as the rotational speed of the rotary screen, stirring interval, centrifuging frequency, and image identification result.

Herein, evaluating the effectiveness of the current enzymatic hydrolysis parameters included: evaluating the efficiency of enzymatic hydrolysis to determine whether the current enzymatic hydrolysis parameters can reach expected efficiency of enzymatic hydrolysis or whether the efficiency of enzymatic hydrolysis was remarkably elevated compared with historical data; evaluating quality of a product to check whether the quality of the product met requirements, including evaluating energy consumption in terms of purity, yield, structure, etc.: analyzing whether energy consumption under the current enzymatic hydrolysis parameters was reasonable, and potential energy-saving space; evaluating operational stability to determine whether the enzymatic hydrolysis process can run stably, without drastic fluctuation or abnormal situations.

Based on evaluation results, the enzymatic hydrolysis control parameters were adjusted.

Herein, the enzymatic hydrolysis control parameters included the rotational speed of the rotary screen, the stirring interval, the centrifuging frequency, etc.

By way of example, an implementation scenario was simulated below according to the above steps.

A fourth batch of corn flour was to be subjected to enzymatic hydrolysis treatment, and data of the first three batches had been collected and a baseline standard was calculated. An enzymatic hydrolysis time baseline of the baseline data of the first three batches was 120 minutes, a rotational speed baseline of the rotary screen was 300 rpm, a stirring interval baseline was 10 minutes, and a centrifuging frequency baseline was every 30 minutes. Real-time monitored data (hypothetical data) of the fourth batch, initial time 0 minutes, current time 60 minutes, 40% of current starch filtration amount, and image identification results showed a moderate to high starch granule concentration. When the starch granule concentration exceeded a certain threshold, stirring was increased and the centrifuging frequency was adjusted from every 30 minutes to every 25 minutes.

The system recorded the current enzymatic hydrolysis progress and temporal data in real time. By comparing the current progress with the baseline, it was found that 40% of the current starch filtration amount within 60 minutes was slightly faster than the baseline progress, and based on the real-time monitored data and the image identification result, the system determined that the current starch granule concentration was relatively high, and stirring should be increased so as to promote enzymatic hydrolysis reaction. As the starch filtration progress was faster than anticipated, the system triggered an early initiation of next centrifugation operation, and adjusted the interval from originally planned 30 minutes to 25 minutes, with the flushing volume remaining unchanged, so as to ensure effective separation of starch without excessive waste of water resources. When the stirring was ceased each time, an image of the mixed liquid in the rotary screen was taken and converted into a grayscale image for processing. The grayscale image was analyzed through the image processing algorithm, so as to confirm the starch filtration progress, which was compared with a preset threshold. If the filtration amount was close to the preset upper limit, it proceeded to the next centrifugation operation. The system continuously monitored the enzymatic hydrolysis progress and dynamically adjusted the control parameters based on the real-time data and the image identification result. If the filtration amount approached the preset upper limit, it proceeded to the next centrifugation operation.

Simulation result was final enzymatic hydrolysis duration, and due to active control and real-time monitoring, the enzymatic hydrolysis duration of the fourth batch was shortened to 105 minutes, 15 minutes ahead of the baseline time.

It should be noted that, as the enzymatic hydrolysis process is more efficient, activity of the product (such as glucose) is expected to be improved. Specific values need to be determined through experiment, but generally the activity is proportional to the efficiency of enzymatic hydrolysis.

The technical solution in the above example of the present invention at least has the following technical effects or advantages.

By introducing advanced technical means such as data continuous monitoring, baseline establishment, active control, and image identification, intelligent management and optimization of an enzymatic hydrolysis process are achieved. Through real-time data collection and analysis, in conjunction with an image identification technology, the starch filtration progress can be accurately determined, and the enzymatic hydrolysis control parameters are accordingly dynamically adjusted. This closed-loop feedback mechanism not only improves the efficiency of enzymatic hydrolysis, but also ensures stability and controllability of the production process, can significantly shorten the enzymatic hydrolysis time, improve the product activity, and reduce the production cost and energy consumption at the same time.

The above-mentioned are merely for preferred embodiments of the present invention, rather than limiting the present invention. For those skilled in the art, various modifications and changes could be made to the present invention. Any modifications, equivalent substitutions, improvements and so on, within the spirit and principle of the present invention, should be covered within the scope of protection of the present invention.

**Claims**

1. A preparation method for a corn superoxide dismutase, **characterized by** comprising following steps:

   S1. pre-treatment: pulverizing sprouted corn to 80-120 mesh, into the pulverized sprouted corn, adding a pH=7.8, 0.05 mol/L phosphate-buffered saline at a ratio of 1:2-4, and mixing uniformly and grinding to obtain a corn slurry, followed by ultrasonic cell disruption;

S2. enzymatic hydrolysis:

S21. transferring the corn slurry having undergone ultrasonic disruption into a rotary screen, and immersing a lower half of a drum into an enzymatic hydrolysis mixed liquid; starting the rotary screen, so as to enable a corn material to tumble inside the drum while remaining immersed in the enzymatic hydrolysis mixed liquid;

S22. extracting the enzymatic hydrolysis mixed liquid every 10-15 minutes to undergo centrifugal separation, so as to remove suspended starch granules and other impurities;

S23. pouring a centrifuged clear liquid back into the rotary screen, and performing enzymatic hydrolysis cyclically for 2-3 times; and

S24. uniformly stirring the corn slurry having undergone the enzymatic hydrolysis and filtering, collecting a filtrate, and into the filtrate, adding an antioxidant of 0.5%-1.5% of a weight of the filtrate; and

S3. extraction and post-treatment:

S31. sonicating the filtrate to obtain a liquid, and adding any one or more of an activator, a protective agent and a stabilizer into the liquid; and

S32. concentrating and drying: putting the filtrate in a low-temperature vacuum concentrator, and performing evaporation at 22-25 °C for 8-15 hours, so as to obtain a corn superoxide dismutase multienzyme liquid; and uniformly mixing the corn superoxide dismutase multienzyme liquid with silica accounting for 1% of a total weight, placing the mixture into a low-temperature vacuum drying device, and freeze-drying the same, so as to obtain a corn superoxide dismutase multienzyme powder.

2. The preparation method for a corn superoxide dismutase according to claim 1, wherein an axis of the drum of the rotary screen is horizontally provided, a diameter of meshes of the drum is smaller than a particle size of pulverized corn, an opening at one end is configured for charging, and the other end is provided with a discharge port.

3. The preparation method for a corn superoxide dismutase according to claim 1, wherein an amount of the corn slurry added in the S21 is 40%-60% of a volume of the rotary screen; the enzymatic hydrolysis mixed liquid comprises an enzyme complex and a surfactant, wherein the enzyme complex is a mixture of pectinase, cellulase, hemicellulase and protease, an amount of the enzyme complex added is 0.5%-2% of a mass of the corn slurry, and the surfactant is sodium dodecyl sulfonate, and added at 0.5%-0.8% of the mass of the corn slurry; and an enzymatic hydrolysis temperature is 45-55 °C, a pH value is 7.0-8.0, and an enzymatic hydrolysis duration is 40-80 minutes.

4. The preparation method for a corn superoxide dismutase according to claim 1, wherein multiple sets of rotating blades are further installed in the rotary screen; and an object dynamic model is used to simulate object flow during stirring, so as to ensure thorough uniform stirring, wherein a plurality of blades are installed in the rotary screen, initially, a helical blade shape is selected, with a blade width being 10%-20% of a diameter of the rotary screen, and a blade length being slightly less than half of a length of the rotary screen, the blades are arranged in a staggered manner, and an angle of the blades is 30-60°.

5. The preparation method for a corn superoxide dismutase according to claim 1, wherein a transmittance sensor is further installed on a tank body storing the enzymatic hydrolysis mixed liquid, and a mapping relationship model of transmittance with starch concentration is established, comprising:

quantifying the mapping relationship model of relevant transmittance with the starch concentration based on historical data and experimental calibration, and determining analysis and fitting of relevant quantitative data by actual situations;

photographing a state of a material in the rotary screen by a camera below the rotary screen, performing feature extraction and identification, and calculating an immersion ratio of starch granules in the mixed liquid so as to infer enzymatic hydrolysis progress;

performing a grayscale histogram analysis on a collected original image;

subsequently performing a filtration denoising pre-treatment operation, so as to extract features of the starch granules;

applying morphological operations to further clean the image, remove noises, connect adjacent starch-granule regions and extract outline edges of the starch granules;

identifying all starch-granule regions in the image by a connected component labeling algorithm, and calculating an area and shape features of each region;

classifying the extracted features, and distinguishing the starch granules from other impurities;

calculating the number of contact pixels between the starch-granule regions and a mixed-liquid background region to approximate the immersion ratio;

setting a threshold based on acquired data of total starch amount, carrying out step S221, and when the starch concentration exceeds the set threshold, starting a centrifugal separation device; and

after step S221 is ended, carrying out step S231: performing the above operations iteratively until extraction and separation of the superoxide dismutase is completed.

6. The preparation method for a corn superoxide dismutase according to claim 5, wherein after the centrifugal separation device is started in step S221, a solid precipitate and a supernatant are separated, wherein the solid precipitate is primarily undissolved starch granules.

7. The preparation method for a corn superoxide dismutase according to claim 6, wherein the solid precipitate obtained after centrifugation is collected, dried and then weighed using a precision balance, and a mass of the precipitate is recorded, comprising:

S2211. comparing an initial amount of corn flour and a starch content, and calculating an amount of starch to be obtained theoretically;

S2212. calculating a degree of completion of the enzymatic hydrolysis progress by comparing the starch amount in actual precipitate with the theoretical starch amount, so as to obtain a percentage of the degree of completion of the enzymatic hydrolysis progress,

wherein the degree of completion of the enzymatic hydrolysis progress is

$$CR = \frac{Ma}{Ms} * 100\%$$ , wherein Ma is an actual starch amount measured by weighing after drying; and

S2213. analyzing the above steps, so as to evaluate influence of enzymatic hydrolysis conditions comprising temperature, pH value, and enzyme dosage on the degree of completion of the progress.

8. The preparation method for a corn superoxide dismutase according to claim 7, wherein after recording and analyzing the mass of the precipitate, a sensor and a data logging system are further provided, so as to obtain a continuous dynamic data set, comprising variations in various parameters during the enzymatic hydrolysis, and the enzymatic hydrolysis progress and overall temporal data for the same batch of material are monitored and recorded in real time.

9. The preparation method for a corn superoxide dismutase according to claim 8, wherein prior to enzymatic hydrolysis of each batch of material, data of first three batches are collected, a mean value is calculated, and a baseline standard for an enzymatic hydrolysis operation is established, wherein a baseline value formula of the enzymatic hydrolysis operation is as follows:

$$Baseline = \frac{1}{n}\sum_{i=1}^{n} (Data_{batch\_i})$$

where n represents the number of data points for which the mean value is to be calculated, and in this implementation scenario, it refers to the number of the data of the first three batches, $Data_{batch\_i}$ represents a data value of an i-th batch, and $\frac{1}{n}\sum_{i=1}^{n}$

represents a sum of all data values from a first batch to an n-th batch, the formula means that the mean value of the data is calculated by dividing a sum of data values of the first three batches by the number of batches, and the mean value is taken as the baseline value.

10. The preparation method for a corn superoxide dismutase according to claim 9, wherein based on real-time progress of starch filtration in the rotary screen, a rotational speed of the rotary screen and a stirring interval of the mixed liquid are dynamically adjusted, so as to achieve an optimal enzymatic hydrolysis effect;

distribution and concentration of starch in the mixed liquid are analyzed by combining a single filtration amount and a total cumulative amount of starch, and a liquid discharge and flushing frequency of a centrifuge is automatically adjusted;

when the stirring is ceased, the camera captures an image of an upper part of the mixed liquid in the rotary screen, and the image is converted into a grayscale image for analyzing distribution and concentration of starch granules;

grayscale value variations in different regions of the grayscale image are analyzed through an image processing algorithm, and real-time progress and estimated amount of the starch filtration are obtained by determining progress and mass of the starch filtration;

effectiveness of current enzymatic hydrolysis parameters is evaluated by comprehensively analyzing data comprising the rotational speed of the rotary screen, the stirring interval, centrifuging frequency, and image identification result; and
based on evaluation results, enzymatic hydrolysis control parameters are adjusted.

FIG. 1

Further installing a transmittance sensor on a tank body storing the enzymatic hydrolysis mixed liquid, and establishing a mapping relationship model of transmittance with starch concentration

↓

Photographing a state of the material in the rotary screen by a camera below the rotary screen, performing feature extraction and identification, and calculating an immersion ratio of starch granules in the mixed liquid so as to infer enzymatic hydrolysis progress

↓

Performing a grayscale histogram analysis on a collected original image

↓

Subsequently performing a filtration denoising pre-treatment operation, so as to extract features of the starch granules

↓

Applying morphological operations to further clean the image, remove noises, connect adjacent starch-granule regions and extract outline edges of the starch granules

↓

Identifying all starch-granule regions in the image by a connected component labeling algorithm, and calculating an area and shape features of each region

↓

Classifying the extracted features, and distinguishing the starch granules from other impurities

**FIG. 2**

# EUROPEAN SEARCH REPORT

**Application Number**

EP 25 19 9419

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 101 892 204 A (ZUOHONG LI) 24 November 2010 (2010-11-24) * claims 1-7 * | 1-10 | INV. C12P21/02 C12N9/08 C12M1/00 |
| Y | CN 205 323 340 U (ZHEJIANG XUYUAN DUZHONG BIOLOGICAL TECH CO LTD) 22 June 2016 (2016-06-22) * paragraphs [0010], [0020]; claim 1; figure 4 * | 1-10 | C12M1/06 C12M1/26 C12M1/34 C12Q3/00 |
| A,D | CN 112 646 789 A (WANG LEI) 13 April 2021 (2021-04-13) * paragraph [0006]; claims 1-9 * | 1-10 | |
| A | CN 1 087 678 A (UNIV SHENYANG AGRICULTURAL [CN]) 8 June 1994 (1994-06-08) * paragraphs [0012], [0017], [0018] * | 1-10 | |
| A | CN 1 587 395 A (CHEN XIN [CN]) 2 March 2005 (2005-03-02) * claim 1 * | 1-10 | |
| A | CN 206 063 847 U (WENZHOU CHINZ MACHINERY CO LTD) 5 April 2017 (2017-04-05) * claims 1-3; figures 1-2 * | 1-10 | TECHNICAL FIELDS SEARCHED (IPC) C12N C12P C12M C12Q B01D |

The present search report has been drawn up for all claims

| Place of search | Date of completion of the search | Examiner |
|---|---|---|
| The Hague | 16 February 2026 | Blanco Parte, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 25 19 9419

This annex lists the patent family members relating to the patent documents cited in the above-mentioned European search report.
The members are as contained in the European Patent Office EDP file on
The European Patent Office is in no way liable for these particulars which are merely given for the purpose of information.

16-02-2026

| Patent document cited in search report | | Publication date | Patent family member(s) | Publication date |
|---|---|---|---|---|
| CN 101892204 | A | 24-11-2010 | NONE | |
| CN 205323340 | U | 22-06-2016 | NONE | |
| CN 112646789 | A | 13-04-2021 | NONE | |
| CN 1087678 | A | 08-06-1994 | NONE | |
| CN 1587395 | A | 02-03-2005 | NONE | |
| CN 206063847 | U | 05-04-2017 | NONE | |

EPO FORM P0459

For more details about this annex : see Official Journal of the European Patent Office, No. 12/82

REFERENCES CITED IN THE DESCRIPTION

**Patent documents cited in the description**

- CN 202011634220